# EUROPEAN PATENT APPLICATION

(11) **EP 4 714 366 A1**
(43) Date of publication of application: **25.03.2026**
(21) Application number: 25202505.1
(22) Date of filing: 16.09.2025
(51) Int. Cl.: A61B 10/00, G01N 21/00, E03D 11/13, B01L 3/00

(54) **MICROFLUIDIC DEVICE FOR TOILET**

(30) Priority: 24.09.2024 US 202463698109 P
(71) Applicant: Outsense Diagnostics Ltd., Tel-Aviv 4791011 (IL)
(72) Inventor: KAPP-BARNEA, Yaara, 4480500 Nirit (IL)
(74) Representative: Boult Wade Tennant LLP

(57) **Abstract**

A system is provided for use with a bodily emission emitted into a toilet bowl of a toilet, and a passageway in fluid communication with the toilet bowl such that the toilet is flushable via the passageway. The system includes a microfluidic device (52), which comprises at least one microfluidic channel (54) and is placeable within the passageway such that a portion of the bodily emission fills the microfluidic channel (54) when the toilet is flushed. A microscopic imaging device (58) is configured to acquire at least one microscopic image of the portion of the bodily emission while the portion is in the microfluidic channel (54). Other applications are also described.

## Description

### CROSS-REFERENCE TO RELATED APPLICATIONS

The present application claims priority from U.S. Provisional Patent Application No. 63/698,109 to Kapp-Barnea, filed September 24, 2024, entitled "Toilet with Microfluidic Device," which is incorporated herein by reference.

### TECHNICAL FIELD

Embodiments of the present disclosure generally relate to the analysis of bodily emissions, such as urine and stool. Specifically, some embodiments of the present disclosure relate to toilet-based systems for analyzing such emissions.

### BACKGROUND

Various microscopic particles (or "entities") found in urine or stool can indicate an underlying infection, disease, or other physiological condition. For example, microcrystals in the urine of a subject can indicate that the subject is dehydrated or that the subject's blood contains an abnormally high level of a substance such as ethylene glycol, calcium (e.g., calcium carbonate, calcium phosphate, or calcium oxalate), uric acid, or bilirubin. Various types of bacteria, fungi, or other microorganisms in the stool of a subject can indicate an infection.

### SUMMARY

To identify a microscopic particle in a bodily emission such as urine or stool, it is often helpful to image the bodily emission under a microscope. However, safely collecting a sample of the bodily emission for such imaging is often not practical.

To address this challenge, embodiments of the present disclosure provide a microscopic imaging system that, advantageously, does not require any manual collection of a sample. The system is for use with a passageway, such as a siphon, configured for fluid communication with the toilet bowl of a toilet into which the bodily emission is emitted, such that the toilet is flushable via the passageway. The system includes a microfluidic device, which includes at least one microfluidic channel and is disposed within the passageway such that a portion of the bodily emission fills the microfluidic channel when the toilet is flushed. The system further includes a microscopic imaging device configured to acquire at least one microscopic image of the portion of the bodily emission while the portion is in the microfluidic channel.

In some embodiments, the system further includes a tube configured to connect the microfluidic channel to the tank of the toilet. Following the flushing of the toilet, some water from the tank flows, via the tube, through the microfluidic channel, thereby clearing the portion of the bodily emission from the microfluidic channel. Alternatively or additionally, the system further includes a vibrating element configured to vibrate the microfluidic device, following the flushing of the toilet, so as to clear the portion of the bodily emission from the microfluidic channel.

Typically, the system further includes a processor configured to process any acquired microscopic images so as to derive properties of the bodily emission, such as a count and classification of a particle in the portion of the bodily emission, from the microscopic images. In some embodiments, the system further includes at least one sensor, such as a light sensor and/or microphone, configured to sense the bodily emission before the toilet is flushed. The processor is further configured to derive additional properties of the bodily emission from the output of this sensor. For some applications, the processor is configured to derive a macroscopic property of the sample, such as the concentration of the bodily emission in the toilet bowl, from the output of this sensor. For some application, the processor is configured to communicate an output that combines microscopic properties with macroscopic properties. For example, in some embodiments, based on the count of the particle, which is derived from the microscopic imaging, and the concentration of the bodily emission, which is derived from the macroscopic sensing, the processor computes and outputs an estimated concentration of the particle in the bodily emission.

There is therefore provided, in accordance with some applications of the present disclosure, a system for use with a bodily emission emitted into a toilet bowl of a toilet, and a passageway in fluid communication with the toilet bowl such that the toilet is flushable via the passageway, the system including:
a microfluidic device, which includes at least one microfluidic channel and is placeable within the passageway such that a portion of the bodily emission fills the microfluidic channel when the toilet is flushed; and
a microscopic imaging device, configured to acquire at least one microscopic image of the portion of the bodily emission while the portion is in the microfluidic channel.

In some embodiments, the system further includes the toilet, and wherein the passageway is integrated into the toilet.

In some embodiments, the passageway includes a siphon of the toilet.

In some embodiments, an inlet of the microfluidic channel is configured to be exposed to air except for during a flushing of the toilet.

In some embodiments, the passageway is configured for installation downstream from the toilet.

In some embodiments,
the system includes the passageway and a wall of the passageway is shaped to define an opening, and
the microfluidic device is mounted into the opening and includes a transparent cover that faces the microscopic imaging device.
In some embodiments, the microscopic imaging device is configured to spectrally image the portion of the bodily emission while the portion is in the microfluidic channel.

In some embodiments,
the toilet includes a tank configured to refill with water following a flushing of the toilet, and
the system further includes a tube configured to connect the microfluidic channel to the tank such that, following the flushing of the toilet, some of the water flows from the tank, via the tube, through the microfluidic channel, thereby clearing the portion of the bodily emission from the microfluidic channel.

In some embodiments, the system further includes a valve connected to the tube and configured to open for a predefined duration following the flushing of the toilet.

In some embodiments, the system further includes a vibrating element configured to vibrate the microfluidic device, following the flushing of the toilet, so as to clear the portion of the bodily emission from the microfluidic channel.

In some embodiments, the system further includes a processor configured to process the microscopic image so as to derive, from the image, a count and classification of a particle in the portion of the bodily emission.

In some embodiments, the system further includes:
at least one sensor, configured to sense the bodily emission before the toilet is flushed, and to output a signal responsively to the sensing; and
a processor, configured to:
   derive one or more first properties of the bodily emission from the microscopic image by processing the image,
   derive one or more second properties of the bodily emission from the signal, and communicate an output that combines the first properties with the second properties.

In some embodiments, the sensor includes a light sensor.

In some embodiments, the sensor includes a microphone.

In some embodiments, the one or more second properties include a concentration of the bodily emission in the toilet bowl.

In some embodiments, the one or more first properties include a count and classification of a particle in the portion of the bodily emission, and the output includes a concentration of the particle in the bodily emission.

There is further provided, in accordance with some embodiments of the present disclosure, the following examples, at least some of which are independent aspects of the present disclosure.

According to an independent aspect, the present disclosure includes Example 1:
Example 1. A system for use with a bodily emission emitted into a toilet bowl of a toilet, the system comprising:
   a microfluidic device, which comprises at least one microfluidic channel and is configured for positioning within or downstream from the toilet such that a portion of the bodily emission fills the microfluidic channel;
   a microscopic imaging device, configured to acquire at least one microscopic image of the portion of the bodily emission while the portion is in the microfluidic channel;
   at least one sensor, configured to sense the bodily emission outside the microfluidic channel, and to output a signal responsively to the sensing; and
   a processor, configured to:
      derive one or more first properties of the bodily emission from the microscopic image by processing the image,
      derive one or more second properties of the bodily emission from the signal, and
      communicate an output that combines the first properties with the second properties.
Example 2. The system according to Example 1, wherein the sensor comprises a light sensor.
Example 3. The system according to Example 1, wherein the sensor comprises a microphone.
Example 4. The system according to Example 1, wherein the microscopic imaging device is configured to spectrally image the portion of the bodily emission while the portion is in the microfluidic channel.
Example 5. The system according to any one of Examples 1-4, wherein the one or more first properties include a count and classification of a particle in the portion of the bodily emission.
Example 6. The system according to Example 5, wherein the one or more second properties include a concentration of the bodily emission in the toilet bowl.
Example 7. The system according to Example 6, wherein the output includes a concentration of the particle in the bodily emission.

According to an independent aspect, the present disclosure includes Example 1:
Example 8. A system for use with a bodily emission emitted into a toilet bowl of a toilet, the toilet including a tank configured to refill with water following a flushing of the toilet, and for use with a passageway in fluid communication with the toilet bowl such that the toilet is flushable via the passageway, the system comprising:
   a microfluidic device, which comprises at least one microfluidic channel and is placeable within the passageway such that a portion of the bodily emission fills the microfluidic channel when the toilet is flushed; and
   a tube configured to connect the microfluidic channel to the tank such that, following the flushing of the toilet, some of the water flows from the tank, via the tube, through the microfluidic channel, thereby clearing the portion of the bodily emission from the microfluidic channel.
Example 9. The system according to Example 8, further comprising a valve connected to the tube and configured to open for a predefined duration following the flushing of the toilet.
Example 10. The system according to Example 8, further comprising a vibrating element configured to vibrate the microfluidic device, following the flushing of the toilet, so as to clear the portion of the bodily emission from the microfluidic channel.
Example 11. The system according to Example 8, wherein an inlet of the microfluidic channel is configured to be exposed to air except for during the flushing of the toilet.
Example 12. The system according to any one of Examples 8-11, wherein the passageway is configured for installation downstream from the toilet.
Example 13. The system according to any one of Examples 8-11, wherein the system further comprises the toilet, and wherein the passageway is integrated into the toilet.
Example 14. The system according to Example 13, wherein the passageway comprises a siphon of the toilet.

The present disclosure will be more fully understood from the following detailed description of embodiments thereof, taken together with the drawings, in which:

### BRIEF DESCRIPTION OF THE DRAWINGS

Fig. 1 is a schematic illustration of a system for analyzing a bodily emission, in accordance with some embodiments of the present disclosure;
Fig. 2 is a schematic illustration of a toilet, in accordance with some embodiments of the present disclosure; and
Fig. 3 is a block diagram showing components of a sensor module, in accordance with some embodiments of the present disclosure.

### DETAILED DESCRIPTION

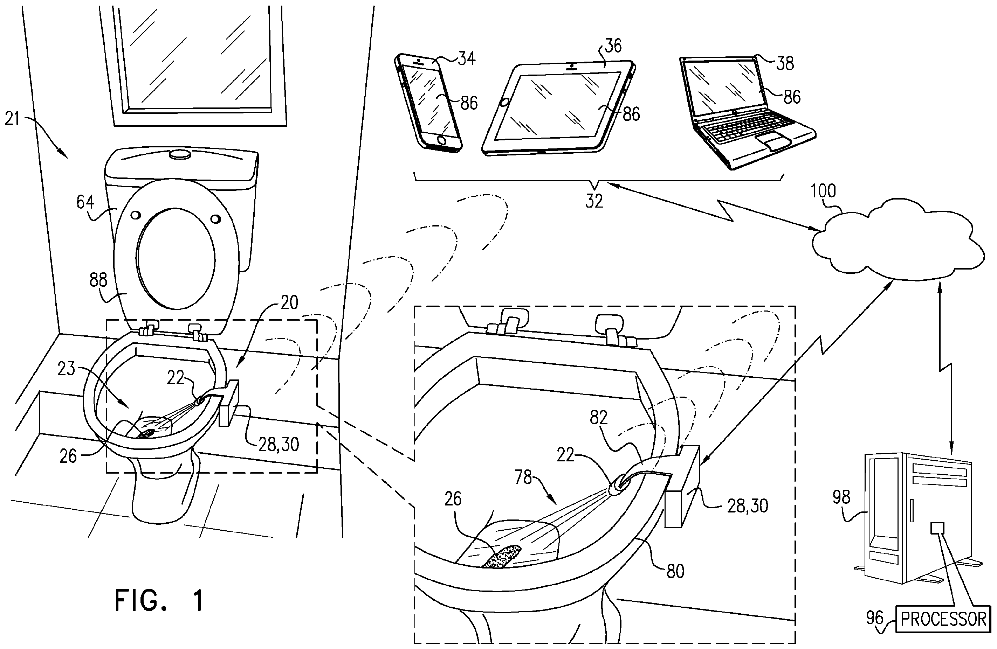

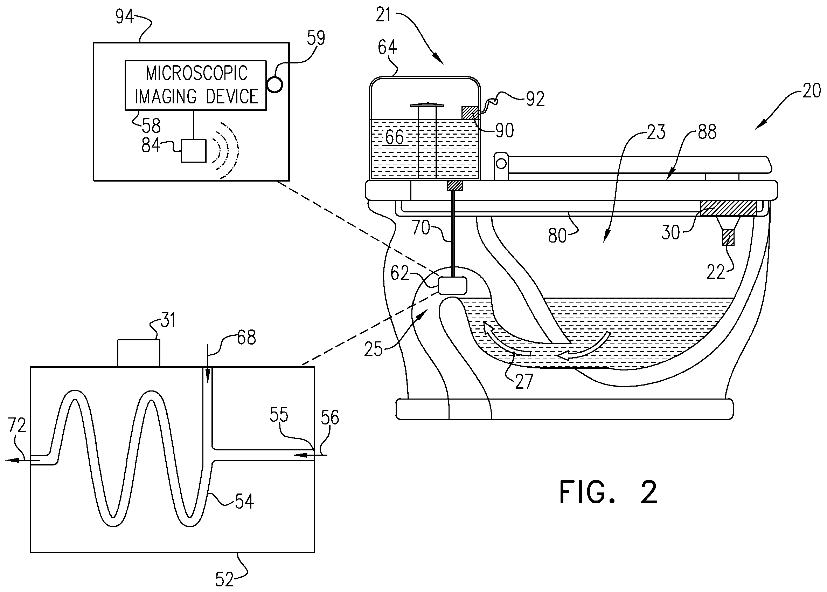

Reference is initially made to Fig. 1, which is a schematic illustration of a system 21 for analyzing a bodily emission 26, such as urine or stool, emitted by a subject into a toilet bowl 23 of a toilet 20, in accordance with some embodiments of the present disclosure. Reference is additionally made to Fig. 2, which is a schematic illustration of toilet 20, in accordance with some embodiments of the present disclosure.

System 21 is typically for use with a passageway 25 that is in fluid communication with toilet bowl 23 such that toilet 20 is flushable via passageway 25, as indicated in Fig. 2 by flushing indicators 27. Typically, in addition to toilet bowl 23, toilet 20 includes a seat 88 and/or a tank 64 configured to empty into the toilet bowl during the flushing of the toilet and to refill with water 66 following the flushing. In general, toilet 20 can be a siphonic toilet, a washdown toilet, or any other type of toilet.

In some embodiments, as illustrated in Fig. 2, passageway 25 is integrated into toilet 20. For example, in some embodiments, passageway 25 includes a siphon of the toilet. In other embodiments, passageway 25 is configured for installation downstream from the toilet, e.g., behind a wall or under the floor of a restroom in which the toilet is installed.

As shown in Fig. 2, system 21 includes a microfluidic device 52, which includes at least one microfluidic channel 54 and is placeable within passageway 25 such that a portion of the bodily emission (while mixed with water from the toilet bowl) fills microfluidic channel 54, e.g., via capillary action, as indicated by a flow indicator 56, when the toilet is flushed. For example, in some embodiments, a wall of passageway 25 is shaped to define an opening 62, and microfluidic device 52 is mounted into opening 62. Typically, the inlet 55 of the microfluidic channel is exposed to air except for during the flushing of the toilet, such that bodily emission 26 readily flows into the microfluidic channel when the toilet is flushed. For example, in some embodiments in which toilet 20 is siphonic, the microfluidic device is positioned within the siphon of the toilet such that inlet 55 is above the resting water level in toilet bowl 23.

For some applications, the wall of the passageway is shaped to define opening 62, and microfluidic device 52 is reversibly mounted into opening 62, such that the microfluidic device is replaceable periodically.

In some embodiments, channel 54 is closed, such that fluid can enter or exit the channel only via dedicated openings such as inlet 55. In other embodiments, channel 54 is open.

System 21 further includes a microscopic imaging module 94 including a microscopic imaging device 58, which is configured to acquire at least one microscopic image of the portion of the bodily emission while the portion is in microfluidic channel 54. Typically, microscopic imaging device 58 includes a light source 59, such as one or more light-emitting diodes, configured to emit light, an imaging sensor, such as a charge coupled device or complementary metal-oxide semiconductor, configured to sense reflections of the emitted light and/or light that is fluoresced in response to the emitted light, and one or more magnifying lenses. Typically, for embodiments in which microfluidic device 52 is mounted into opening 62, microscopic imaging module 94 is located behind the wall of passageway 25, and microfluidic device 52 includes a transparent cover that faces the microscopic imaging device.

In some embodiments, microscopic imaging device 58 is configured to spectrally image the portion of the bodily emission while the portion is in the microfluidic channel. For example, in some embodiments, light source 59 includes multiple emitters configured to emit light at different respective ranges of wavelengths, and/or the imaging sensor includes multiple spatially-arranged filters configured to transmit different respective ranges of wavelengths. Advantageously, the spectral imaging facilitates the detection of particles in the bodily emission.

In some embodiments, light source 59 illuminates the microfluidic channel with ultraviolet light, such as light having a wavelength between 300 and 400 nm (e.g., around 365 nm), so as to elicit fluorescence, which is typically in the visible or near infrared range. Alternatively or additionally, light source 59 illuminates the microfluidic channel with wideband illumination (e.g., illumination having a band of wavelengths between 400 and 800 nm), which typically reflects in the visible range. Alternatively or additionally, light source 59 illuminates the microfluidic channel in the near infrared range, e.g., with light having a wavelength between 650 and 1050 nm. Alternatively or additionally, the microscopic imaging device images the portion of the bodily emission, even without illuminating the microfluidic channel, based on light emitted by the bodily emission. (In such embodiments, the microscopic imaging device does not necessarily include a light source.) For example, in some embodiments, the microscopic imaging device acquires thermal images in the near infrared range.

In some embodiments, system 21 further includes a tube 70 configured to connect microfluidic channel 54 to tank 64 such that, following the flushing of the toilet, some of water 66 flows from tank 64, via tube 70, through microfluidic channel 54, as indicated by a flow indicator 68. The water thus clears the portion of the bodily emission from the microfluidic channel, as indicated by another flow indicator 72. Typically, in such embodiments, system 21 further includes a valve 74 connected to tube 70 (e.g., to the inlet or outlet of the tube) and configured to open for a predefined duration following the flushing of the toilet.

Alternatively or additionally, system 21 further includes a vibrating element 31 configured to vibrate microfluidic device 52, following the flushing of the toilet, so as to clear the portion of the bodily emission from microfluidic channel 54. In some embodiments, vibrating element 31 includes a pair of electrodes, and/or a piezoelectric element, that contacts the microfluidic device, and hence vibrates the microfluidic device when an electric current is passed through the vibrating element. Alternatively or additionally, vibrating element 31 includes an ultrasonic emitter configured to emit ultrasonic waves that vibrate the microfluidic device.

More generally, microfluidic device 52 can be configured for positioning anywhere within or downstream from toilet 20 such that the portion of the bodily emission (typically, while mixed with toilet water) fills microfluidic channel 54.

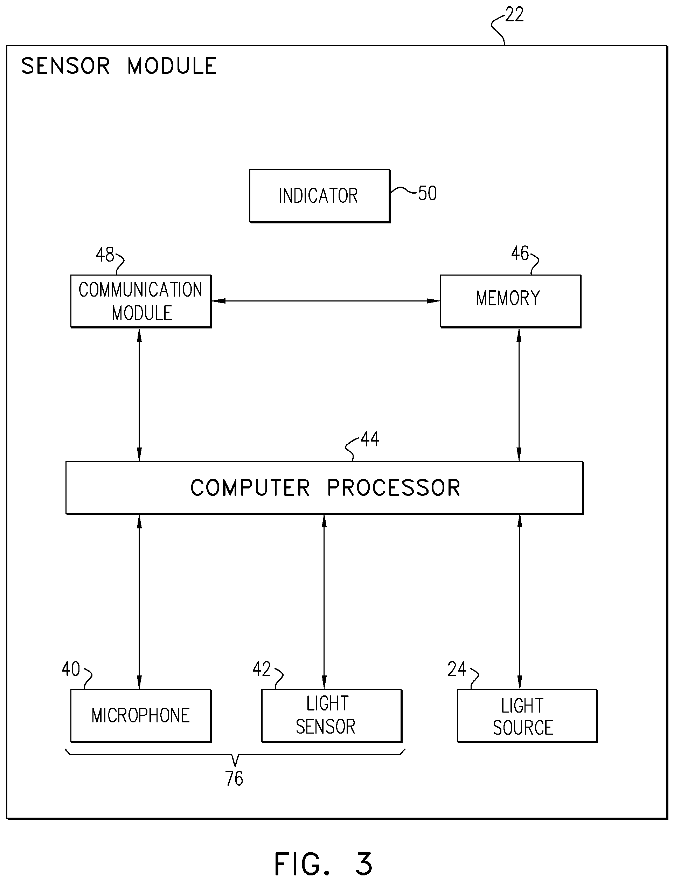

Reference is now additionally made to Fig. 3, which is a block diagram showing components of sensor module 22, in accordance with some embodiments of the present disclosure.

In some embodiments, system 21 further includes one or more sensors 76, each of which is configured to sense bodily emission 26 outside microfluidic channel 54, typically before the toilet is flushed (e.g., while the bodily emission is emitted and/or while the bodily emission is in the toilet bowl), and to output a signal responsively to the sensing. Typically, sensors 76 are contained in a sensor module 22, which typically includes a water-resistant housing.

For example, in some embodiments, system 21 includes a microphone 40 configured to sense the sound of the emission of the bodily emission. Alternatively or additionally, system 21 includes a light sensor 42, such as an imaging sensor and/or a spectral sensor for example, configured to sense light that is emitted, reflected, or fluoresced from bodily emission 26 while the bodily emission is emitted and/or while the bodily emission is in toilet bowl 23. Typically, in such embodiments, system 21 further includes a light source 24 configured to emit light 78 (schematically illustrated in Fig. 1) at the bodily emission, thereby illuminating the bodily emission for light sensor 42. Typically, the face of the sensor module underneath light source 24 and light sensor 42 is transparent.

In some embodiments, sensor module 22 is connected to the mains electricity. Alternatively or additionally, as shown in Fig. 1, sensor module 22 is powered wiredly or wirelessly by a power source 28 (e.g., a battery pack) disposed within a housing 30. For example, in some embodiments, as shown in Fig. 1, sensor module 22 is connected to housing 30 by a connecting arm 82, which is mounted onto the rim 80 of toilet bowl 23 such that the sensor module is inside the toilet bowl and the housing is outside the toilet bowl. (In some such embodiments, the sensor module is at least partly submerged within the toilet bowl.) In other embodiments, the sensor module is integrated into toilet bowl 23.

In some embodiments, microscopic imaging module 94 is also powered by power source 28, e.g., via a wired connection. In other embodiments, module 94 is powered by a separate power source.

Typically, sensor module 22 includes a computer processor 44 configured to control, and/or receive input from, other components of the sensor module such as light source 24 and/or sensors 76. In some embodiments, processor 44 is further configured to instruct microscopic imaging module 94 to begin imaging the bodily emission in the microfluidic channel in response to one or more inputs indicating that the toilet has been flushed. Examples of such inputs include a signal from microphone 40, an image acquired by light sensor 42, and a signal from a dedicated flush sensor 90 coupled to the flush mechanism 92 of the toilet, which is shown in Fig. 2. Typically, the processor instructs the microscopic imaging module to begin the imaging following a predefined duration from the start of the flush. Typically, processor 44 is further configured to receive output, such as microscopic images, from microscopic imaging module 94.

In some embodiments, to facilitate the exchange of communication with microscopic imaging module 94, sensor module 22 is wiredly connected to microscopic imaging module 94. Alternatively, sensor module 22 further includes a communication module 48, including a wireless communication interface, and microscopic imaging module 94 further includes another wireless communication interface 84 (Fig. 2), which is configured to exchange communication with communication module 48.

Similarly, in some embodiments, processor 44 is further configured to open valve 74, and/or to activate vibrating element 31, in response to one or more inputs indicating that the toilet has been flushed. For example, in some embodiments, following a predefined duration from the start of the flush - which is typically the duration needed for tank 64 to almost finish refilling - the processor opens valve 74 and/or activates vibrating element 31. Subsequently, after another predefined duration, the processor closes the valve and/or deactivates the vibrating element. The processor may be connected to valve 74 and/or vibrating element 31 over any suitable wired or wireless connection.

In some embodiments, sensor module 22 includes a memory 46 in which processor 44 is configured to store data, such as microscopic images received from the microscopic imaging module. In some such embodiments, memory 46 includes a removable memory card, such as a Secure Digital card.

Typically, as shown in Fig. 1, system 21 further includes a processor 96 configured to process the microscopic images acquired by microscopic imaging device 58 so as to derive one or more properties of the bodily emission from the images. In some embodiments, these properties include the count and classification (i.e., type) of a particle in the portion of the bodily emission in microfluidic channel 54. For example, by processing the images, processor 96 may classify the particle as a urine microcrystal, a bacteria, a fungus, or another type of microorganism (e.g., based on a predefined library that delineates the manner in which each type of microorganism emits, reflects, and/or fluoresces light), and further estimate the count of the particle.

Typically, for embodiments in which the system includes sensors 76, processor 96 is further configured to process any signals output by the sensors so as to derive additional properties of the bodily emission from the signals. For some applications, the additional properties include one or more macroscopic properties of the sample. In some embodiments, the properties include the type(s) of bodily emission in the toilet bowl, the volume of urine in the toilet bowl, the mass and Bristol scale classification of stool in the toilet bowl, and/or the concentration of the bodily emission in the toilet bowl. For some applications, the processor is further configured to communicate an output that combines the first properties with the second properties by virtue of including both sets of properties and/or including another property of the bodily emission derived from both sets of properties. For example, in some embodiments, the output includes the concentration (i.e., number of particles per unit volume) of each particle type of interest in the bodily emission, the concentration being derived from the count of the particle in the microscopic sample and the concentration of the bodily emission in the toilet bowl (e.g., the concentration of urine in the toilet bowl).

In some embodiments, processor 96 belongs to a device 98, such as a cloud server, that is remote from toilet 20. In such embodiments, typically, processor 44 of sensor module 22 is configured to communicate data acquired by microscopic imaging module 94 and sensors 76, over at least one network 100 (e.g., a Wi-Fi network and/or the Internet), to device 98, via communication module 48.

In some embodiments, as shown in Fig. 1, system 21 further includes at least one device 32 belonging, for example, to the subject or to the subject's healthcare provider. Processor 96 is configured to communicate the aforementioned output to device 32, e.g., over network 100, and device 32 includes a display 86 configured to display the output. In some embodiments, device 32 includes a smartphone 34, a tablet computer 36, or a laptop computer 38.

In some embodiments, in addition to the properties of the bodily emission, the output from processor 96 may include additional details and/or instructions. For example, based on the analysis of the bodily emission, processor 96 may output a message indicating whether a condition, such as thrush or diarrhea, is bacterial or mycotic. Alternatively or additionally, the processor may output a suggested treatment plan, which may include the intake of antibiotics or antimycotics. Alternatively or additionally, the processor may instruct the subject to seek medical care.

In some embodiments, one or more other processors perform at least some of the functionality of processor 96 described hereinabove. For example, in some embodiments, processor 44 and/or a processor of device 32 performs the analysis of data from microscopic imaging module 94 and/or sensors 76.

In some embodiments, sensor module 22 includes an indicator 50 configured to indicate to the subject when a sample has been successfully imaged, and/or when data has been successfully transmitted to a remote device. In some embodiments, indicator 50 includes a visual indicator, such as a light-emitting diode. Alternatively or additionally, indicator 50 includes an audio indicator (for example, a speaker that is configured to emit a beep). The indicator typically interacts with other components of the sensor module, such as the computer processor and/or the communication module.

In general, each of the processors described herein may be embodied as a single processor, or as a cooperatively networked or clustered set of processors. The functionality of the processor may be implemented solely in hardware, e.g., using one or more fixed-function or general-purpose integrated circuits, Application-Specific Integrated Circuits (ASICs), and/or Field-Programmable Gate Arrays (FPGAs). Alternatively, this functionality may be implemented at least partly in software. For example, the processor may be embodied as a programmed processor including, for example, a central processing unit (CPU) and/or a Graphics Processing Unit (GPU). Program code, including software programs, and/or data may be loaded for execution and processing by the CPU and/or GPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description.

## Claims

1. A system for use with a bodily emission emitted into a toilet bowl of a toilet, and a passageway in fluid communication with the toilet bowl such that the toilet is flushable via the passageway, the system comprising:
a microfluidic device (52), which comprises at least one microfluidic channel (54) and is placeable within the passageway such that a portion of the bodily emission fills the microfluidic channel (54) when the toilet is flushed; and
a microscopic imaging device (58), configured to acquire at least one microscopic image of the portion of the bodily emission while the portion is in the microfluidic channel (54).

2. The system according to claim 1, wherein the system further comprises the toilet, and wherein the passageway is integrated into the toilet.

3. The system according to claim 2, wherein the passageway comprises a siphon of the toilet.

4. The system according to claim 1, wherein an inlet of the microfluidic channel (54) is configured to be exposed to air except for during a flushing of the toilet.

5. The system according to claim 1, wherein the passageway is configured for installation downstream from the toilet.

6. The system according to claim 1,
wherein the system comprises the passageway and a wall of the passageway is shaped to define an opening, and
wherein the microfluidic device is mounted into the opening and comprises a transparent cover that faces the microscopic imaging device (58).

7. The system according to claim 1, wherein the microscopic imaging device (58) is configured to spectrally image the portion of the bodily emission while the portion is in the microfluidic channel (54).

8. The system according to claim 1,
wherein the toilet includes a tank configured to refill with water following a flushing of the toilet, and
wherein the system further comprises a tube (70) configured to connect the microfluidic channel to the tank such that, following the flushing of the toilet, some of the water flows from the tank, via the tube, through the microfluidic channel (54), thereby clearing the portion of the bodily emission from the microfluidic channel.

9. The system according to claim 8, further comprising a valve (74) connected to the tube (70) and configured to open for a predefined duration following the flushing of the toilet.

10. The system according to claim 1, further comprising a vibrating element (31) configured to vibrate the microfluidic device (52), following the flushing of the toilet, so as to clear the portion of the bodily emission from the microfluidic channel (54).

11. The system according to any one of claims 1-10, further comprising a processor configured to process the microscopic image so as to derive, from the image, a count and classification of a particle in the portion of the bodily emission.

12. The system according to any one of claims 1-10, further comprising:
at least one sensor (40, 42), configured to sense the bodily emission before the toilet is flushed, and to output a signal responsively to the sensing; and
a processor (44), configured to:
derive one or more first properties of the bodily emission from the microscopic image by processing the image,
derive one or more second properties of the bodily emission from the signal, and
communicate an output that combines the first properties with the second properties.

13. The system according to claim 12, wherein the sensor comprises a light sensor (42) and/or a microphone (40).

14. The system according to claim 12, wherein the one or more second properties include a concentration of the bodily emission in the toilet bowl.

15. The system according to claim 15, wherein the one or more first properties include a count and classification of a particle in the portion of the bodily emission, and wherein the output includes a concentration of the particle in the bodily emission.
